# EUROPEAN PATENT APPLICATION

(11) **EP 4 840 463 A1**
(43) Date of publication of application: **18.11.2026**
(21) Application number: 25738920.5
(22) Date of filing: 10.01.2025
(51) Int. Cl.: A61K 45/00, A61P 11/00, A61P 43/00

(54) **THERAPEUTIC AGENT FOR IDIOPATHIC PULMONARY FIBROSIS**

(30) Priority: 11.01.2024 JP 2024002318
(71) Applicant: The Jikei University, Tokyo 105-8461 (JP)
(72) Inventor: FUJITA, Yu, Tokyo 105-8461 (JP); ARAYA, Jun, Tokyo 105-8461 (JP); WATANABE, Naoaki, Tokyo 105-8461 (JP)
(74) Representative: Icosa
(86) International application number: PCT/JP2025/000613
(87) International publication number: WO 2025/150553

(57) **Abstract**

A therapeutic agent for idiopathic pulmonary fibrosis, comprising a PAK inhibitor as an active ingredient.

## Description

### TECHNICAL FIELD

The present invention relates to a therapeutic agent for idiopathic pulmonary fibrosis.

### BACKGROUND ART

Idiopathic pulmonary fibrosis (IPF) is a chronic progressive interstitial lung disease of unknown cause that is characterized by refractory and progressive pulmonary fibrosis and has a very poor prognosis, with a 5-year survival rate of about 20% to 40%. IPF is caused by excessive production of an extracellular matrix by myofibroblasts induced by chronic inflammation or tissue injury, and it is known that the formation of immature fibroblastic foci is particularly deeply involved in the pathogenesis.

Persistent abnormal activation of lung myofibroblasts is mediated by various proteins such as transforming growth factor (TGF), platelet-derived growth factor, and fibroblast growth factor. The persistent abnormal activation of lung myofibroblasts is a major event in development and progression of IPF.

An IPF microenvironment is composed of highly heterogeneous cells that dynamically interact with each other. Continuous interaction between fibrotic tissue and its microenvironment plays an important role in the development, the progression, and response to various treatments of fibrosis. In regions between fibrotic tissue and a morphologically normal lung, fibroblasts of various sizes, usually referred to as early fibroblastic foci (FF), are typically found. FF is a major pathogenic lesion in IPF and is composed of activated fibroblasts and myofibroblasts, which are major effector cells responsible for dysregulated extracellular matrix (ECM) deposition in various fibrotic states. Consistent with the idea that fibrosis spreads from FF to non-involved alveoli, these structures are sites where myofibroblasts accumulate during pathological ECM deposition. In fact, the larger the number of FFs is more closely associated with worsening of symptoms in IPF patients.

A genomic landscape of IPF has been partially clarified using conventional sequencing strategies such as bulk whole exome sequencing and bulk RNA sequencing (RNA-seq) (see, for example, Non Patent Document 1).

### Citation List

### Non Patent Document

Non Patent Document 1: Peyser R, MacDonnell S, Gao Y, Cheng L, Kim Y, Kaplan T, et al. Defining the Activated Fibroblast Population in Lung Fibrosis Using Single-Cell Sequencing. Am J Respir Cell Mol Biol. 2019;61(1):74-85.

### SUMMARY OF INVENTION

### Technical Problem

However, in silico analysis has been performed only using a mixture of normal tissue with components other than FF in a single tissue. Single-cell RNA sequencing (scRNA-seq) is an unprecedented tool for analyzing heterogeneity of IPF lungs at a single-cell level and developing a comprehensive gene expression atlas, but it lacks spatial information of single cells. Spatial information is essential for comprehensively understanding the progression of IPF. Localized tissue heterogeneity is a characteristic pathological feature of fibrosis in the human lung.

Therefore, it is urgent to develop a sequencing technology for determining cell type-specific spatial profiles. In this regard, the approach of a digital spatial transcriptome provides gene expression information with spatial resolution and is useful for obtaining a general understanding of tissue formation and the pathogenesis of diseases. Therefore, by utilizing this methodology, it is possible to contribute to the understanding of fibrogenesis in IPF lungs in various conditions.

There is still no fundamental treatment for IPF, and its development is urgent. Therefore, an object of the present invention is to provide a therapeutic agent for idiopathic pulmonary fibrosis based on a new mechanism of action by utilizing the above-described methodology.

### Solution to Problem

The present invention is as follows.
[1] A therapeutic agent for idiopathic pulmonary fibrosis, comprising a PAK inhibitor as an active ingredient.
[2] A therapeutic agent for idiopathic pulmonary fibrosis, comprising a PAK2 inhibitor as an active ingredient.
[3] The therapeutic agent for idiopathic pulmonary fibrosis according to [1] or [2], wherein the therapeutic agent is intended for use in patients having WNT5A+ CTHRC1+ myofibroblasts in lung tissue.
[4] A composition for treating idiopathic pulmonary fibrosis, comprising: the therapeutic agent for idiopathic pulmonary fibrosis according to [1] or [2]; and a pharmaceutically acceptable carrier.
[5] A diagnostic agent for diagnosing idiopathic pulmonary fibrosis, the diagnostic agent comprising: a primer set for amplifying at least one molecule selected from the group consisting of POSTN, THBS2, MMP11, CTHRC1, COL5A2, MMP14, MXRA5, ENC1, COL12A1, MMP7, COL8A1, ASPN, TGFBI, COL5A1, SPON2, COL7A1, ADAM12, CREB3L1, PMEPA1, and VCAN in a sample, and/or a probe that binds to the molecule or an amplification product thereof, and an antibody that binds to the molecule.
[6] A method for detecting a diagnostic marker for idiopathic pulmonary fibrosis, the method comprising: detecting an amount of at least one molecule selected from the group consisting of POSTN, THBS2, MMP11, CTHRC1, COL5A2, MMP14, MXRA5, ENC1, COL12A1, MMP7, COL8A1, ASPN, TGFBI, COL5A1, SPON2, COL7A1, ADAM12, CREB3L1, PMEPA1, and VCAN in a sample derived from a subject.

### Advantageous Effects of Invention

According to the present invention, it is possible to provide a therapeutic agent for idiopathic pulmonary fibrosis based on a new mechanism of action.

### BRIEF DESCRIPTION OF DRAWINGS

[FIG. 1] An experimental workflow for spatial transcriptome analysis and scRNA-seq analysis of a human IPF lung. For the spatial transcriptome analysis, nine lung sections derived from three patients were used, and for the scRNA-seq analysis, three IPF lungs and three control lungs were used.
[FIG. 2] A spatial visualization image of fibroblastic foci (FF) annotation based on expression levels of COL1A1, POSTN, and CTHRC1 shown in each panel. A representative enlarged image of a divided box region shows hematoxylin and eosin (H&E) staining that highlights FF in IPF lung tissue.
[FIG. 3] A UMAP visualization image of an annotated interstitial scRNA-seq dataset from three IPF lung samples and three control lung samples, labeled with detailed cell types.
[FIG. 4] A violin plot showing expression of selected marker genes in each cluster.
[FIG. 5] A UMAP visualization image of the scRNA-seq dataset of FIG. 3 classified by origin from a normal lung or an IPF lung.
[FIG. 6] A bar graph comparing a composition of lung interstitial cell types between healthy subjects and IPF patients.
[FIG. 7] A result of pseudotime trajectory analysis predicting different differentiation trajectories of interstitial cells between healthy subjects and IPF patients.
[FIG. 8] An immunohistochemical image of PDGFRA, CTHRC1, and WNT5A in fibroblastic foci in IPF lung tissue.
[FIG. 9] A result of spatial visualization of FF and advanced fibrotic lesion (DF) annotation along an estimated cell type distribution on a Visium section. In a representative zoom-in image of a boundary box region showing hematoxylin and eosin (H&E) staining, FF and DF in IPF lung tissue are highlighted.
[FIG. 10] A result of 3D PCA mapping using 97 extracted FF spots and 412 extracted DF spots.
[FIG. 11] A dot plot showing what types of interstitial cells are present in FF, DF, and SMC regions selected on Visium by integrated analysis of single-cell data and spatial analysis data.
[FIG. 12] A spatial transcriptome spot (patient JKPF2-1) of an FF index.
[FIG. 13] A bar graph representing a ratio of a high FF index population and a low FF index population in an interstitial cell type.
[FIG. 14] A result of upstream regulator prediction by IPA for 746 differentially expressed genes between FF and DF. The top 10 upstream regulators predicted to be "activated" are shown.
[FIG. 15] A violin plot showing an average expression level of the top 10 upstream regulators detected in FIG. 14 in each cell type.
[FIG. 16] A visualization result of a PAK target gene signature in an interstitial population displayed on UMAP.
[FIG. 17] A visualization result of an FF index signature in an interstitial population displayed on UMAP.
[FIG. 18] A violin plot showing expression of PAK2 in high FF index interstitial cells and low FF index interstitial cells.
[FIG. 19] A spatial visualization result of a PAK target gene signature and an estimated cell type distribution along the FF/DF annotation on a Visium section.
[FIG. 20] A dot plot showing expression of PAK family genes compared between interstitial cells of a normal lung sample and an IPF lung sample.
[FIG. 21] A result of hematoxylin and eosin (H&E) staining and immunohistochemical staining for PAK1, PAK2, and PAK3 in fibroblastic foci of IPF lung tissue.
[FIG. 22] A result showing an effect of siRNA-mediated PAK1/2 knockdown on TGF-β-induced myofibroblast differentiation in lung fibroblasts. An upper part shows a schematic diagram of a protocol used in an in vitro experiment. A lower part shows a representative immunoblot analysis result showing amounts of PAK1, PAK2, type I collagen, and β-actin in PAK1- or PAK2-knockdown lung fibroblasts after 48 hours of TGF-β1 (2 ng/mL) treatment.
[FIG. 23] A result of qPCR showing expression of WNT5A in PAK1- or PAK2-knockdown human lung fibroblasts after 48 hours of TGF-β1 (2 ng/mL) treatment.

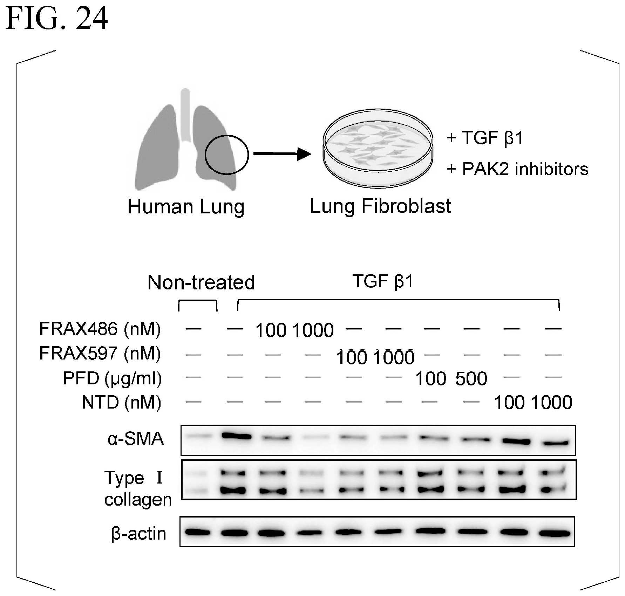

[FIG. 24] A result showing an effect of PAK inhibitors FRAX486 and FRAX597 on TGF-β-induced myofibroblast differentiation in lung fibroblasts. An upper part shows a schematic diagram of a protocol used in an in vitro experiment. A lower part shows a representative immunoblot analysis result showing amounts of α-smooth muscle actin (α-SMA), type I collagen, and β-actin in lung fibroblasts treated with FRAX486 (100 nM and 1,000 nM) and FRAX597 (100 nM and 1,000 nM) for 48 hours in the presence of TGF-β1 (2 ng/mL). Pirfenidone (PFD) and nintedanib (NTD) were used as positive controls for inhibiting myofibroblast differentiation.
[FIG. 25] A quantitative immunoblot analysis result showing amounts of type I collagen and α-SMA.
[FIG. 26] An immunoblot analysis result showing the amounts of PAK2 and its downstream signals in TGF-β-induced myofibroblasts treated with FRAX486 (1,000 nM) and FRAX597 (1,000 nM) for 48 hours in the presence of TGF-β1.
[FIG. 27] A result showing an effect of a PAK2 inhibitor on IPF lung fibroblasts. A representative immunoblot analysis result showing amounts of α-SMA, type I collagen, and β-actin in IPF lung fibroblasts treated with FRAX486 (1,000 nM) and FRAX597 (1,000 nM) for 48 hours. A protein sample was recovered from cells 48 hours after start of the treatment.
[FIG. 28] A quantitative immunoblot analysis result showing amounts of type I collagen and α-SMA in IPF lung fibroblasts.
[FIG. 29] A schematic protocol used for FRAX486 treatment of bleomycin-induced pulmonary fibrosis mice.
[FIG. 30] A violin plot of Pak2 between bleomycin-induced lung fibroblasts and control fibroblasts.
[FIG. 31] An immunoblot analysis result confirming enhancement of PAK2 phosphorylation in lung tissue of a BLM-induced fibrosis model.
[FIG. 32] A graph showing a change in body weight observed after bleomycin administration. The body weight on day 0 before start of the treatment was set to 100. Values in the graph represent mean ± SEM. **P < 0.01. Control: n = 10; bleomycin control: n = 10; bleomycin + FRAX486: n = 10.
[FIG. 33] A quantification result of hydroxyproline in a left lung of each mouse. Each dot represents data from one animal. ***P < 0.001, ****P < 0.0005.
[FIG. 34] A representative image of H&E staining and Masson's trichrome staining of lung sections from each group of treated mice.
[FIG. 35] A quantification result of fibrosis using an Ashcroft score. Each dot represents data from one animal. ****P < 0.0005.
[FIG. 36] A representative p-PAK2 IHC staining result of lung sections from each group of treated mice. Scale bar: 100 µm.

### DESCRIPTION OF EMBODIMENTS

### [Therapeutic agent for idiopathic pulmonary fibrosis]

As will be described later in Examples, the inventors have found antifibrotic properties of PAK2 inhibitors from the viewpoint of IPF treatment. In one embodiment, the present invention provides a therapeutic agent for idiopathic pulmonary fibrosis comprising a PAK inhibitor as an active ingredient.

The p21-activated kinase (PAK) family is composed of serine/threonine kinases that regulate various cellular activities. To date, six PAK isoforms (PAK1 to PAK6) have been identified.

PAK1 is essential for the proliferation of solid tumors, and it is known that the excessive activation or overexpression of PAK1 causes diseases such as cancer, hypertension, diabetes, and Alzheimer's disease.

Examples of the amino acid sequence of PAK1 include a sequence registered in GenBank Accession No. AAA65441 in human PAK1. Examples of the gene sequence of PAK1 include a sequence registered in GenBank Accession No. U24152 in human PAK1.

The PAK1 inhibitor is not limited as long as it is a substance that inhibits signal transduction by PAK1 or inhibits a signal transduction pathway caused by PAK1, and examples thereof include a nucleic acid, a protein, and a low-molecular-weight compound. Examples of the substance that directly acts on PAK1 include a low-molecular-weight compound, a protein, an antibody, and an aptamer. Examples of the substance that suppresses the expression of the gene encoding PAK1 include siRNA and an antisense oligonucleotide.

Examples of the low-molecular-weight compound that inhibits the kinase activity of PAK1 include IPA-3 (1,1'-dithiodi-2-naphthol), AG-1478 (N-(3-chlorophenyl)-6,7-dimethoxy-4-quinazolinamine), FRAX597 (6-[2-chloro-4-(1,3-thiazol-5-yl)phenyl]-8-ethyl-2-[4-(4-methylpiperazin-1-yl)anilino]pyrido[2,3-d]pyrimidin-7-one), FRAX486 (6-(2,4-dichlorophenyl)-8-ethyl-2-[[3-fluoro-4-(1-piperazinyl)phenyl]amino]pyrido[2,3-d]pyrimidin-7(8H)-one), and PF-3758309 ((S)-N-(2-(dimethylamino)-1-phenylethyl)-6,6-dimethyl-3-((2-methylthieno[3,2-d]pyrimidin-4-yl)amino)-4,6-dihydropyrrolo[3,4-c]pyrazol-5(1H)-carboxamide). IPA-3 is a low-molecular-weight allosteric inhibitor of PAK1.

PAK2 is widely distributed throughout the body and is an important effector of the Rho family of low-molecular-weight GTPases associated with reorganization of the cytoskeleton.

Furthermore, PAK2 controls various biological processes such as tumor formation, cellular senescence, and promotion of organismal aging. In particular, PAK2 is an activated receptor for TGF-β and is a downstream component of AKT. The activation of PAK2 is essential for the profibrotic action of TGF-β. Furthermore, PAK2 is a non-canonical SMAD effector of TGF-β in renal interstitial fibrogenesis, and the overexpression thereof induces renal fibrogenesis. In some studies, the relationship between PAK2 and fibrosis has been clarified in hepatic stellate cells and peritoneal mesothelial cells, but the involvement of PAK2 in the pathogenesis of IPF has not been sufficiently studied.

Examples of the amino acid sequence of PAK2 include a sequence registered in GenBank Accession No. AAA65442 in human PAK2. Examples of the gene sequence of PAK2 include a sequence registered in GenBank Accession No. U24153 in human PAK2.

The PAK2 inhibitor is not limited as long as it is a substance that inhibits signal transduction by PAK2 or inhibits a signal transduction pathway caused by PAK2, and examples thereof include a nucleic acid, a protein, and a low-molecular-weight compound. Examples of the substance that directly acts on PAK2 include a low-molecular-weight compound, a protein, an antibody, and an aptamer. Examples of the substance that suppresses the expression of a gene encoding PAK2 include siRNA and an antisense oligonucleotide.

Examples of the low-molecular-weight compound that inhibits the kinase activity of PAK2 include FRAX486 (6-(2,4-dichlorophenyl)-8-ethyl-2-[[3-fluoro-4-(1-piperazinyl)phenyl]amino]pyrido[2,3-d]pyrimidin-7(8H)-one), FRAX597 (6-[2-chloro-4-(1,3-thiazol-5-yl)phenyl]-8-ethyl-2-[4-(4-methylpiperazin-1-yl)anilino]pyrido[2,3-d]pyrimidin-7-one), FRAX1036, PF-3758309 ((S)-N-(2-(dimethylamino)-1-phenylethyl)-6,6-dimethyl-3-((2-methylthieno[3,2-d]pyrimidin-4-yl)amino)-4,6-dihydropyrrolo[3,4-c]pyrazol-5(1H)-carboxamide), IPA-3 (1,1'-dithiodi-2-naphthol), AG-1478 (N-(3-chlorophenyl)-6,7-dimethoxy-4-quinazolinamine), 2-aminopyrido[2,3-d]pyrimidin-7(8H)-one, 1H-thieno[3,2-c]pyrazole, 3-amino-tetrahydropyrrolo[3,4-c]pyrazole, N4-(1H-pyrazol-3-yl)pyrimidine-2,4-diamine, and N2-bicyclic indolyl, indazolyl, and benzimidazolyl derivatives of N4-(1H-pyrazol-3-yl)pyrimidine-2,4-diamine.

The PAK inhibitor used in the therapeutic agent for idiopathic pulmonary fibrosis according to the present embodiment may be a pan-PAK inhibitor, but is preferably a PAK2 selective inhibitor.

In the therapeutic agent for idiopathic pulmonary fibrosis according to the present embodiment, the PAK inhibitor may be used in the form of a free form or in the form of a pharmaceutically acceptable salt. Further, it may be used in the form of a solvate of a free form or in the form of a solvate of a salt.

The salt is not particularly limited as long as it is a pharmaceutically acceptable salt. Examples of the salt include a hydrochloride, a sulfate, a hydrobromide, a hydroiodide, a phosphate, a nitrate, a benzoate, a methanesulfonate, a 2-hydroxyethanesulfonate, a p-toluenesulfonate, an acetate, a propionate, an oxalate, a malonate, a succinate, a glutarate, an adipate, a tartrate, a maleate, a fumarate, a malate, and a mandelate. The solvate is not particularly limited as long as it is a pharmaceutically acceptable solvate, and examples thereof include a hydrate and an organic solvate.

As will be described later in Examples, since PAK2 is highly expressed in WNT5A+ CTHRC1+ myofibroblasts, it is preferable that the therapeutic agent for idiopathic pulmonary fibrosis according to the present embodiment is intended for use in patients having WNT5A+ CTHRC1+ myofibroblasts in lung tissue.

### [Composition for treating idiopathic pulmonary fibrosis]

In one embodiment, the present invention provides a composition for treating idiopathic pulmonary fibrosis, comprising the above-described therapeutic agent for idiopathic pulmonary fibrosis and a pharmaceutically acceptable carrier.

The composition for treating idiopathic pulmonary fibrosis according to the present embodiment can be administered orally in the form of, for example, a tablet, a coated tablet, a pill, a powder, a granule, a capsule, a liquid preparation, a suspension, or an emulsion, or can be administered parenterally in the form of an inhalation drug, an injection, a suppository, or a topical skin preparation.

As the pharmaceutically acceptable carrier, those typically used in the formulation of a pharmaceutical composition can be used without particular limitation. More specifically, examples thereof include a binder such as gelatin, corn starch, tragacanth gum, or arabic gum; an excipient such as starch or crystalline cellulose; a swelling agent such as alginic acid; a solvent for injection such as water, ethanol, or glycerin; and an adhesive such as a rubber-based adhesive or a silicone-based adhesive. The pharmaceutically acceptable carrier can be used alone or in combination of two or more types thereof.

The composition for treating idiopathic pulmonary fibrosis according to the present embodiment may further include an additive. Examples of the additive include a lubricant such as calcium stearate or magnesium stearate; a sweetener such as sucrose, lactose, saccharin, or maltitol; a flavoring agent such as peppermint or wintergreen oil; a stabilizer such as benzyl alcohol or phenol; a buffer such as a phosphate or sodium acetate; a solubilizing aid such as benzyl benzoate or benzyl alcohol; an antioxidant; and a preservative. The additive can be used alone or in combination of two or more types thereof.

### (Method of administration)

The method of administering the therapeutic agent for idiopathic pulmonary fibrosis or the composition for treating idiopathic pulmonary fibrosis is not particularly limited, and may be appropriately determined according to the symptoms, body weight, age, gender, and the like of the patient. For example, a tablet, a coated tablet, a pill, a powder, a granule, a capsule, a liquid preparation, a suspension, an emulsion, or the like is orally administered. In addition, the injection is intravenously administered alone or in combination with a normal fluid such as glucose or an amino acid, and further, as necessary, is administered intraarterially, intramuscularly, intracutaneously, subcutaneously, or intraperitoneally. The suppository is administered intrarectally. The topical skin preparation is applied, attached, or sprayed to the affected area. The inhalation drug is administered using a device such as a nebulizer, a metered-dose inhaler, or a dry powder inhaler.

### (Dose)

The dose of the therapeutic agent for idiopathic pulmonary fibrosis or the composition for treating idiopathic pulmonary fibrosis varies depending on the symptoms, body weight, age, gender, and the like of the patient, and cannot be unconditionally determined. However, in a case of oral administration, for example, an active ingredient may be administered at 1 µg to 10 g per day, for example, 0.01 to 2,000 mg per day. In addition, in a case of the injection, for example, the active ingredient may be administered at 0.1 µg to 1 g per day, for example, 0.001 to 200 mg per day. In addition, in a case of the suppository, for example, the active ingredient may be administered at 1 µg to 10 g per day, for example, 0.01 to 2,000 mg per day. In addition, in a case of the topical skin preparation, for example, the active ingredient may be administered at 1 µg to 10 g per day, for example, 0.01 to 2,000 mg per day. In a case of the inhalation drug, for example, the active ingredient may be administered at 1 µg to 10 g per single inhalation, for example, 0.01 to 2,000 mg per single inhalation.

### [Another Embodiment]

In one embodiment, the present invention provides a PAK inhibitor, a pharmaceutically acceptable salt thereof, or a solvate thereof for treating idiopathic pulmonary fibrosis.

In one embodiment, the present invention provides a method for treating idiopathic pulmonary fibrosis, comprising administering an effective amount of a PAK inhibitor, a pharmaceutically acceptable salt thereof, or a solvate thereof to patients in need of treatment.

In one embodiment, the present invention provides use of a PAK inhibitor, a pharmaceutically acceptable salt thereof, or a solvate thereof for producing a therapeutic agent for idiopathic pulmonary fibrosis or a composition for treating idiopathic pulmonary fibrosis.

### [Another Embodiment]

In one embodiment, the present invention provides a therapeutic agent for idiopathic pulmonary fibrosis, comprising, as an active ingredient, at least one inhibitor selected from the group consisting of a PRKAA2 inhibitor, a TWF1 inhibitor, a PRKAA1 inhibitor, a DDR2 inhibitor, a PAK2 inhibitor, an ABL2 inhibitor, a DYRK1B inhibitor, an FGFR4 inhibitor, a CDK19 inhibitor, and an ERBB4 inhibitor.

### [Diagnostic agent for diagnostic marker for idiopathic pulmonary fibrosis]

In one embodiment, the present invention provides a diagnostic agent for diagnosing idiopathic pulmonary fibrosis, the diagnostic agent comprising a primer set for amplifying at least one molecule selected from the group consisting of POSTN, THBS2, MMP11, CTHRC1, COL5A2, MMP14, MXRA5, ENC1, COL12A1, MMP7, COL8A1, ASPN, TGFBI, COL5A1, SPON2, COL7A1, ADAM12, CREB3L1, PMEPA1, and VCAN in a sample, and/or a probe that binds to the molecule or an amplification product thereof, and an antibody that binds to the molecule.

Examples of the molecule as a detection target of the diagnostic agent include nucleic acids such as DNA, RNA, and miRNA, and proteins. In a case where the detection target is a nucleic acid, it is preferable that the diagnostic agent includes a primer set for amplifying these molecules, and/or a probe that binds to the molecule or an amplification product thereof. In a case where the detection target is a protein, it is preferable that the diagnostic agent includes an antibody that binds to these molecules.

In the present embodiment, examples of the sample include cells derived from a tissue piece, blood, urine, saliva, sweat, and tissue exudate.

### [Method for detecting a diagnostic marker for idiopathic pulmonary fibrosis]

In one embodiment, the present invention provides a method for detecting a diagnostic marker for idiopathic pulmonary fibrosis, the method comprising detecting an amount of at least one molecule selected from the group consisting of POSTN, THBS2, MMP11, CTHRC1, COL5A2, MMP14, MXRA5, ENC1, COL12A1, MMP7, COL8A1, ASPN, TGFBI, COL5A1, SPON2, COL7A1, ADAM12, CREB3L1, PMEPA1, and VCAN in a sample derived from a subject.

The molecule as a detection target is at least one molecule selected from the group consisting of POSTN, THBS2, MMP11, CTHRC1, COL5A2, MMP14, MXRA5, ENC1, COL12A1, MMP7, COL8A1, ASPN, TGFBI, COL5A1, SPON2, COL7A1, ADAM12, CREB3L1, PMEPA1, and VCAN, and it is preferable that all molecules of POSTN, THBS2, MMP11, CTHRC1, COL5A2, MMP14, MXRA5, ENC1, COL12A1, MMP7, COL8A1, ASPN, TGFBI, COL5A1, SPON2, COL7A1, ADAM12, CREB3L1, PMEPA1, and VCAN are set as detection targets. As will be described later in Examples, the sample can be evaluated by using an average value of the expression levels of these genes as an FF index.

### Examples

Hereinafter, the present invention will be described in more detail with reference to Examples, but the present invention is not limited to these examples.

### [Experimental examples]

### [Spatial transcriptome analysis of human IPF lung tissue]

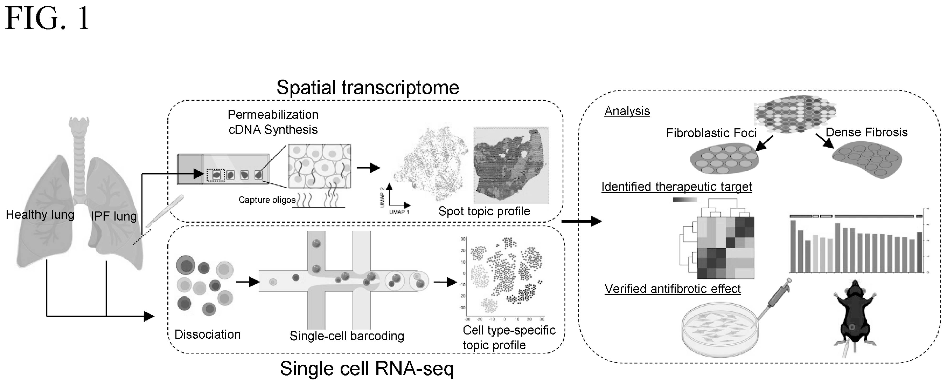

In an experimental flowchart shown in FIG. 1, spatial transcriptome profiling of lung tissues (a total of 9 sections) of three IPF patients was performed. Furthermore, scRNA-seq analysis of lung tissues of three IPF patients and three healthy subjects was performed. As a first step of the spatial transcriptome analysis of the IPF lung, a standard procedure of standardization and visualization using uniform manifold approximation and projection (UMAP) was applied to each section.

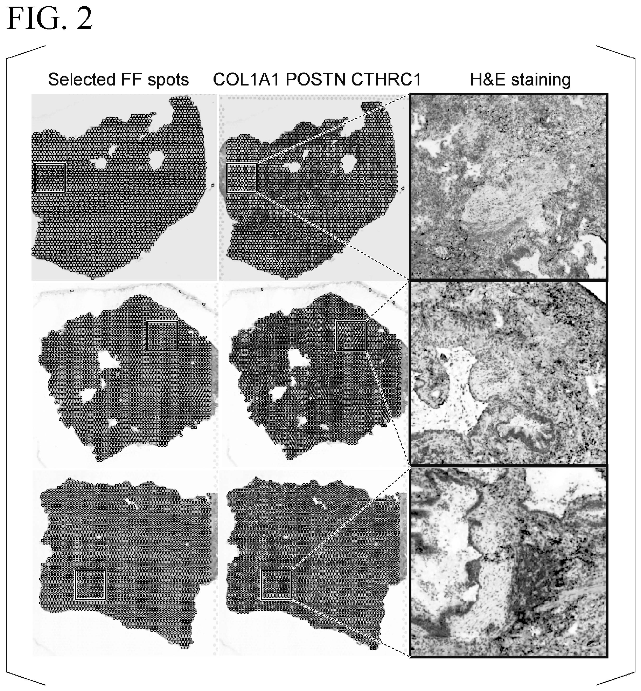

In the IPF lung, FF includes activated fibroblasts and myofibroblasts, and is considered to be a major pathogenic lesion of IPF. In order to characterize the cellular features and molecular features of FF, they were histologically identified in H&E images (see FIG. 2), and Visium spots overlapping these regions were designated as FF spots. The accuracy of the selection of these FF spots was further verified by examining the spatial distribution of known FF markers such as COL1A1, POSTN, and CTHRC1 on each Visium section.

### [Integration of detailed annotation of scRNA-seq data and spatial Visium spots]

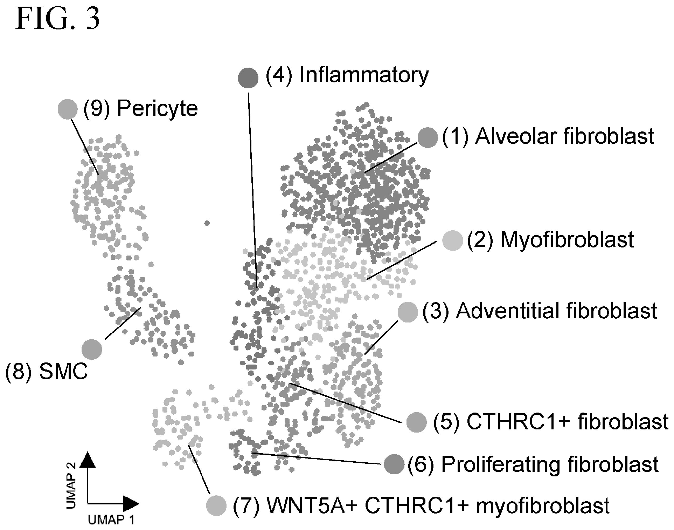

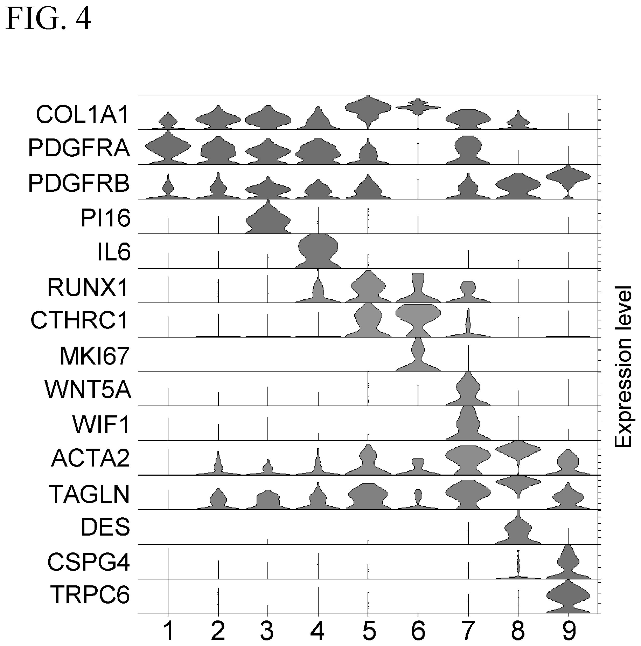

In order to further examine the cell type composition in the fibrotic areas of IPF, the annotation of the interstitial cell types was improved using major markers involved in the pathogenesis of IPF. By UMAP clustering, nine different clusters were identified, including a population of CTHRC1+ fibroblasts known to play an important role in the pathogenesis of IPF (see FIGS. 3 and 4). In particular, an intermediate cell state bridging fibroblasts and smooth muscle cells (SMC)/pericyte clusters was identified and named WNT5A+ CTHRC1+ myofibroblasts. These cells exhibited fibroblast-like characteristics expressing PDGFRA, PDGFRB, and RUNX1, lacked expression of DES, and had specific markers such as WNT5A and WIF1 (see FIG. 4).

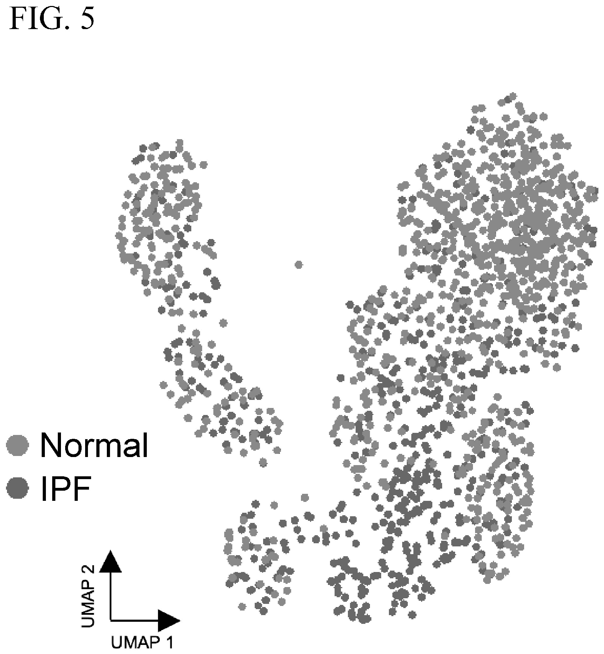

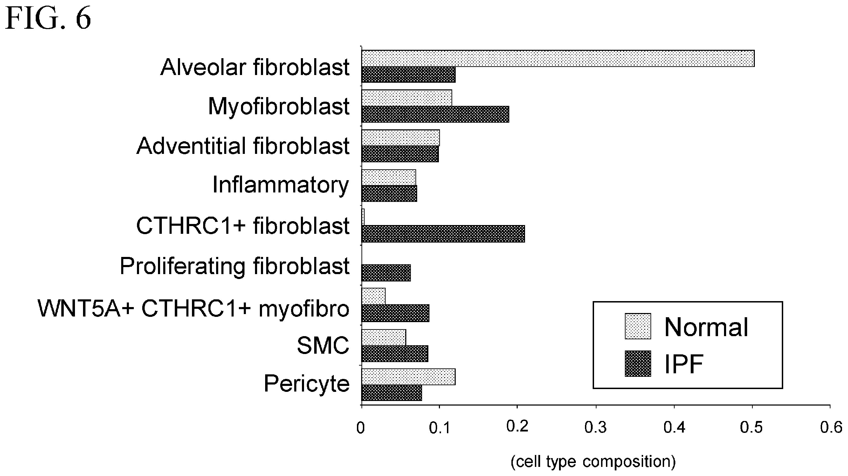

The presence of this WNT5A+ CTHRC1+ myofibroblast population was verified by analyzing publicly available single-cell datasets derived from IPF patients (GSE136831, GSE135893). These WNT5A+ CTHRC1+ cells were detected in a myofibroblast cluster mapped between fibroblasts and SMC clusters on UMAP. Furthermore, the proportions of myofibroblasts, CTHRC1 fibroblasts, and WNT5A+ CTHRC1+ myofibroblasts were significantly increased in IPF patients compared to controls (see FIGS. 5 and 6).

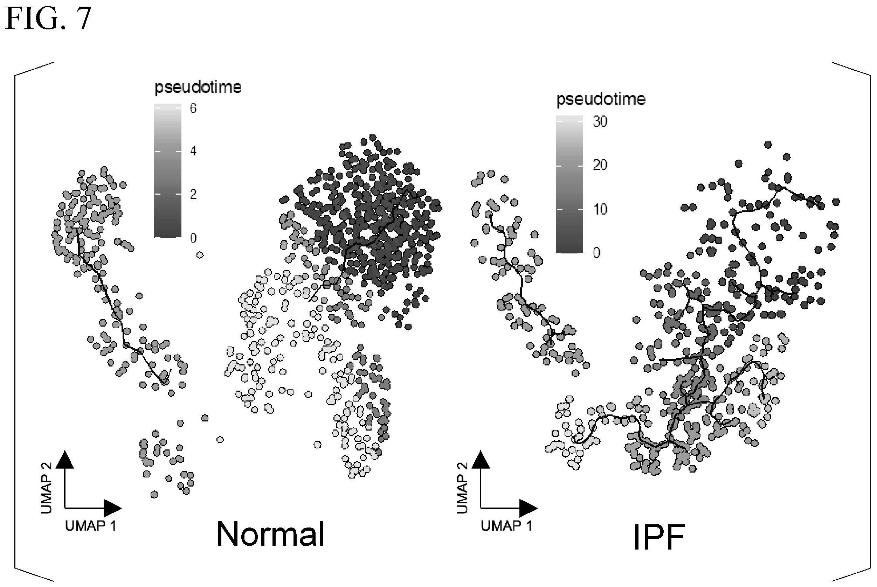

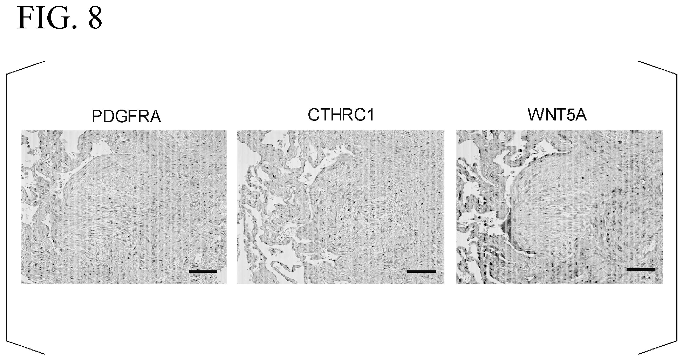

Pseudotime trajectory analysis revealed a fibroblast differentiation pathway characteristic of IPF compared to controls. In lung tissue of the healthy subjects, alveolar fibroblasts mainly differentiate into myofibroblasts. However, in IPF, alveolar fibroblasts follow two different trajectories, differentiation into myofibroblasts or pathogenic CTHRC1+ fibroblasts. The latter population further acquires smooth muscle-like characteristics including increased expression of TAGLN and ACTA2 and transitions to WNT5A+ CTHRC1+ myofibroblasts (see FIG. 7). Immunohistochemistry of human IPF lung tissue confirmed the presence of WNT5A+ CTHRC1+ PDGFRA+ fibroblasts in FF and peripheral DF regions of IPF lung tissue (see FIG. 8).

### [Molecular and cellular differences between FF and DF in human IPF lung]

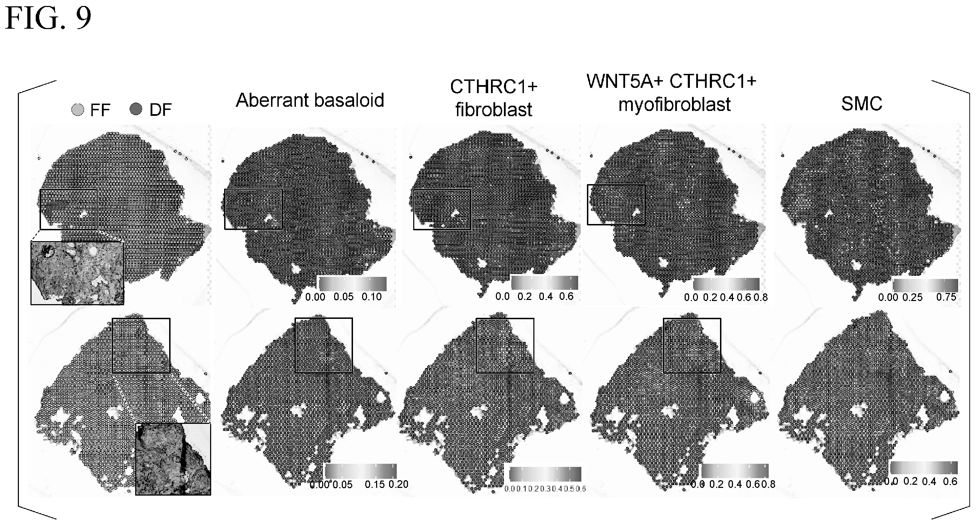

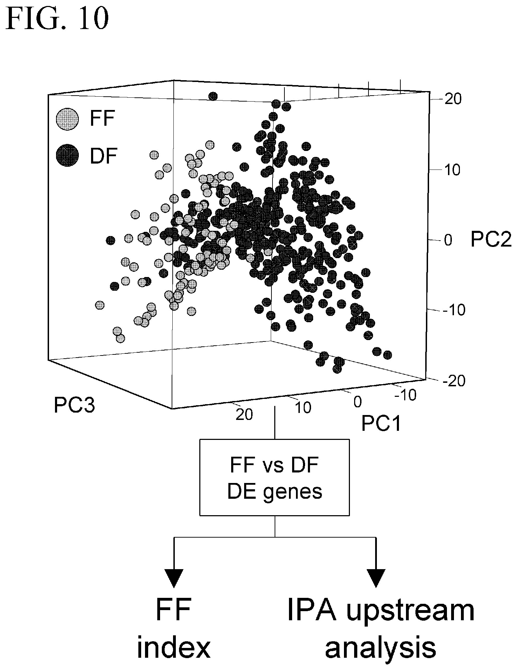

IPF lung tissue exhibits a heterogeneous pattern of fibrosis, similar to the DF region representing fibrotic remodeling in which mature scar tissue disrupts the normal lung structure and impairs gas exchange. Comparing FF and DF is important for understanding the dynamic progression of fibrosis and identifying therapeutic targets for both active fibrogenesis and its transition to fibrotic remodeling. The DF regions surrounding FF, characterized by the abundance of fibroblasts and the absence of SMCs and pericytes, were morphologically identified. Visium spots corresponding to the DF regions were manually curated using deconvolution data from scRNA-seq, and regions with significant contribution from SMCs or pericytes were excluded (see FIG. 9). As a result, 97 FF spots and 412 DF spots were identified from 9 sections. Plotting these spots on a principal component analysis (PCA) map (see FIG. 10) revealed a clear difference in transcriptome profiles between FF and DF.

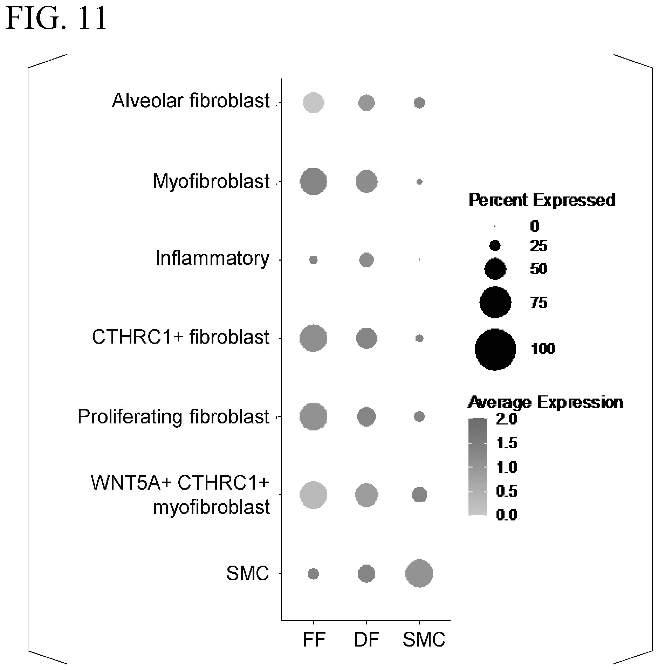

To elucidate the differences in cell type composition, deconvolution of the interstitial scRNA-seq data and Visium data of FF spots and DF spots revealed different patterns of cell distribution in FF and DF. The FF region was mainly occupied by CTHRC1+ fibroblasts, proliferative fibroblasts, and Aberrant Basaloid, but the DF region had a higher cell proportion of WNT5A+ CTHRC1+ myofibroblasts compared to the SMC/pericyte region, further supporting the idea that the transition to WNT5A+ CTHRC1+ myofibroblasts may be partially involved in the mechanism of transition from FF to DF (see FIG. 11). Aberrant Basaloid showed a high level of cell-cell interaction with both WNT5A+ CTHRC1+ myofibroblasts and CTHRC1+ fibroblasts, suggesting the presence of a profibrotic loop that promotes the progression of fibrosis.

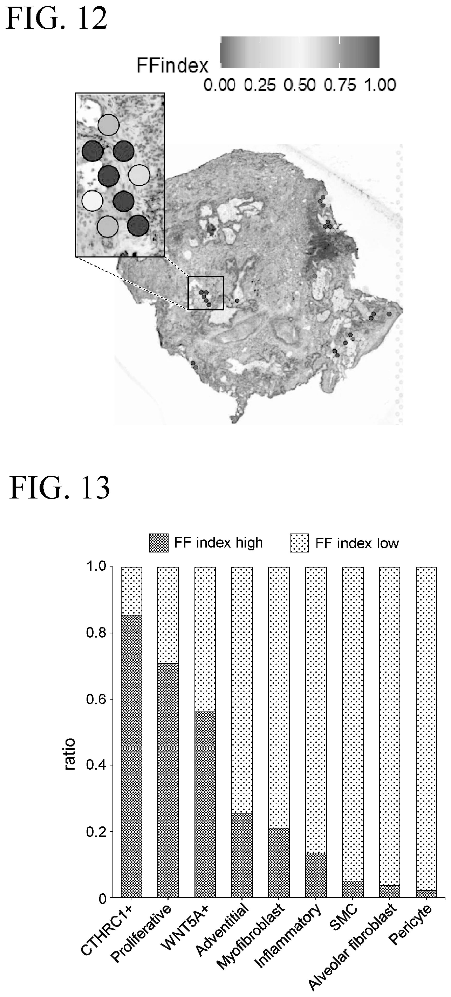

To molecularly detect the fibroblast population in FF, an FF index was developed based on markers identified by spatial analysis and subsequently deconvoluted with the scRNA-seq data. In the control lung samples, fibroblasts with a high FF index were also detected, but the amount of fibroblasts with a high FF index was higher in the IPF samples. Spatially, the FF index effectively visualized fibroblastic foci in the IPF lung section (see FIG. 12). Fibroblasts with a high FF index were mainly composed of pathogenic fibroblasts such as CTHRC1+ fibroblasts, proliferative fibroblasts, and WNT5A+ CTHRC1+ myofibroblasts, whereas fibroblasts with a low FF index were alveolar fibroblasts and the like (see FIG. 13). These findings suggest that fibroblasts with a high FF index may be useful as therapeutic targets for IPF.

### [Identification of PAK2 activation in fibroblastic foci and peripheral DF regions and its therapeutic potential in IPF]

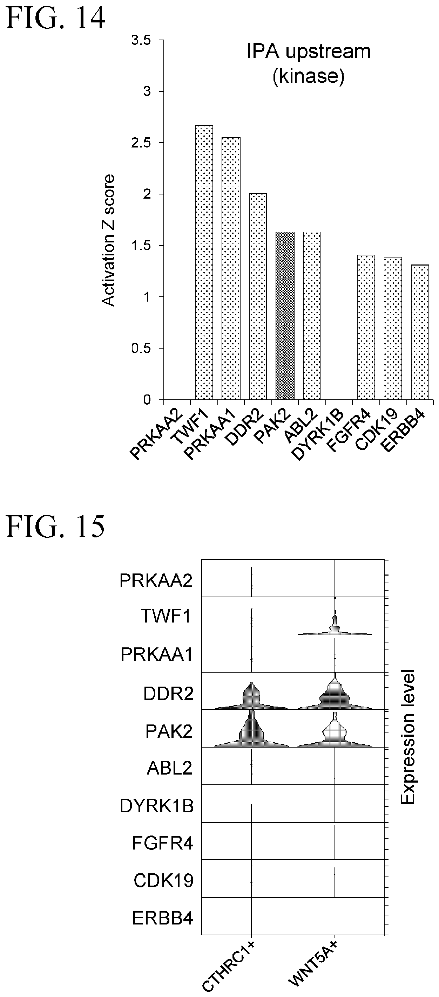

To identify novel therapeutic target molecules for IPF, differentially expressed genes between FF and DF were analyzed. From the results of Ingenuity Pathway Analysis (IPA), the activated "idiopathic pulmonary fibrosis signal transduction pathway" in the FF spots of the IPF lung was clearly demonstrated. The prediction of upstream regulatory factors revealed the activation of several kinases in the FF spots of the IPF lung (see FIG. 14). Among these kinases, PRKAA2, which is a catalytic subunit of AMPK, was considered as a candidate because it can be expected to play a role in regulating the TGF-β-induced myofibroblast differentiation. As part of the search for a novel therapeutic target, PAK2 was focused on because PAK2 is highly expressed in pathogenic CTHRC1+ fibroblasts and WNT5A+ CTHRC1+ myofibroblasts (see FIG. 15) and because a low-molecular-weight inhibitor targeting PAK2 is available.

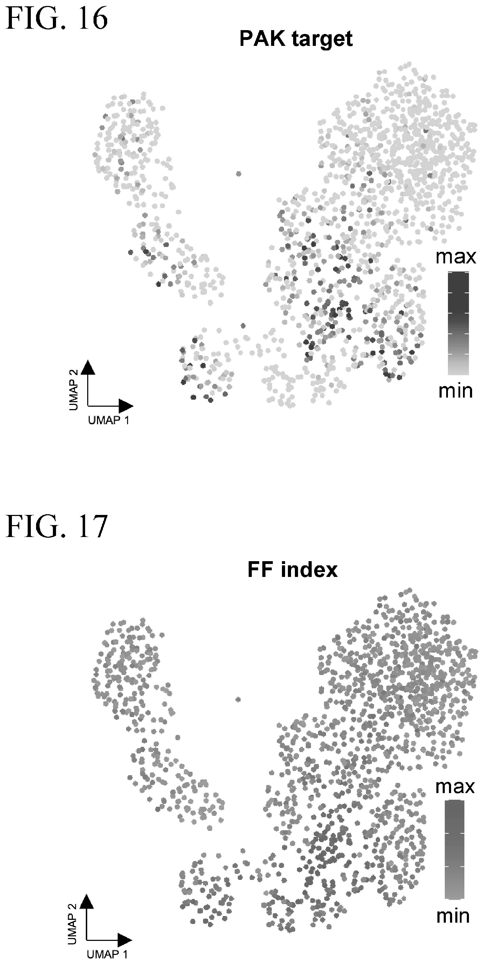

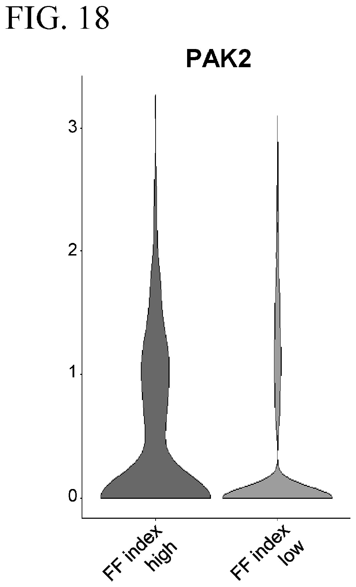

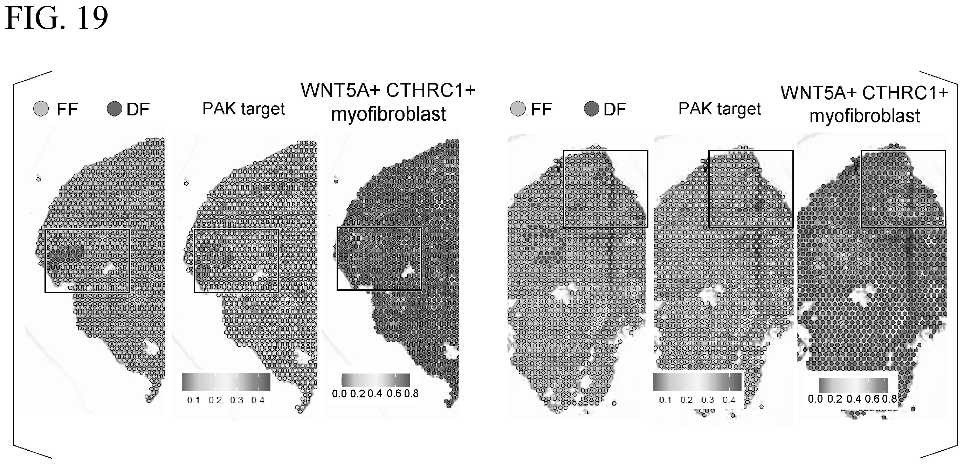

To further examine the activity of PAK2 at the single-cell level, a PAK target gene set was established based on previously reported RNA-seq data. The average expression level of the PAK target signature in each single cell was calculated using a module score algorithm and visualized with UMAP (see FIG. 16). Interestingly, the distribution of cells expressing a high PAK target signature was very similar to the distribution of cells with a high FF index (see FIG. 17), suggesting that PAK target genes are mainly expressed in profibrotic fibroblasts. Furthermore, the expression of PAK2 was significantly higher in fibroblasts with a high FF index compared to fibroblasts with a low FF index (see FIG. 18). Spatial distribution analysis confirmed that the expression of PAK target genes was concentrated around the FF region and the DF region, closely matching the distribution of CTHRC1+ fibroblasts and WNT5A+ CTHRC1+ myofibroblasts, respectively (see FIG. 19).

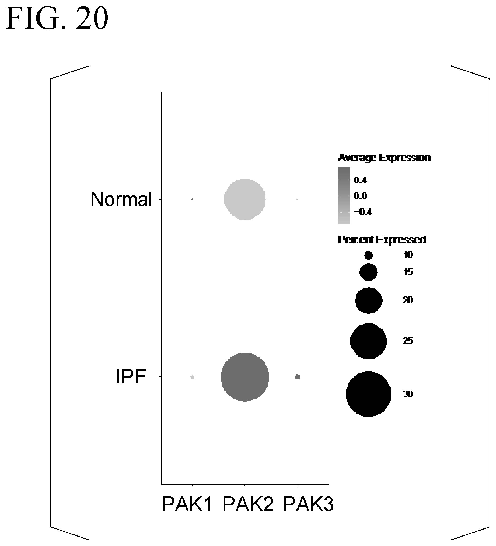

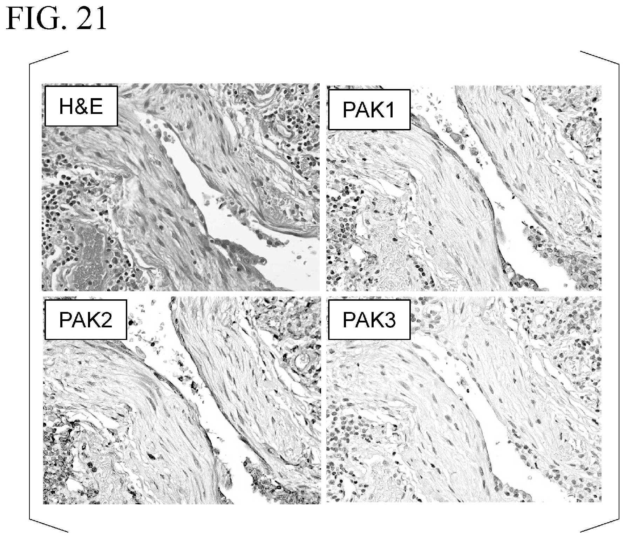

Among the PAK family, PAK2 showed the highest expression in the interstitial cells compared to PAK1 and PAK3, and showed significant enrichment in IPF lung tissue at both the transcriptome level and the protein level (see FIGS. 20 and 21). These findings suggest that PAK2 may be a therapeutic target for IPF by regulating the activity of pathogenic fibroblasts.

### [Role of PAK2 in TGF-β-induced profibrotic differentiation in human lung fibroblasts and its inhibition as a therapeutic target]

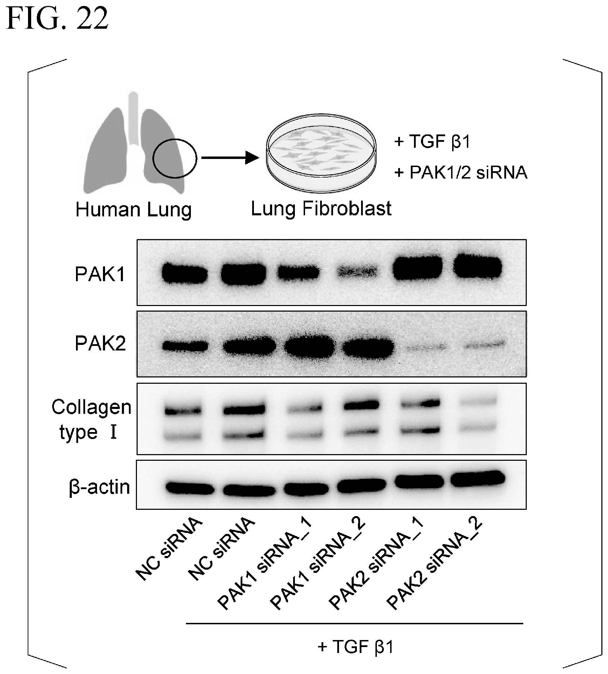

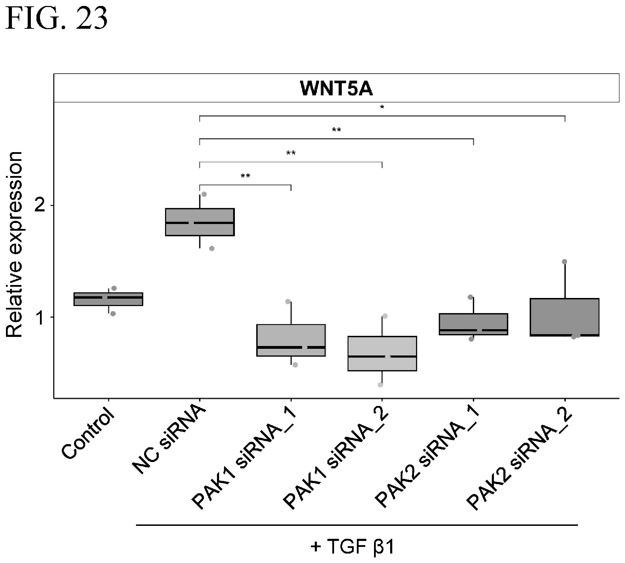

To elucidate the roles of PAK1 and PAK2 in TGF-β-induced fibrotic properties, newly isolated primary human lung fibroblasts were used to perform knockdown of PAK1 and PAK2 by siRNA. The knockdown of both PAK1 and PAK2 efficiently reduced TGF-β-induced ECM production and WNT5A expression (see FIGS. 22 and 23). This is consistent with the results of spatial transcriptome analysis.

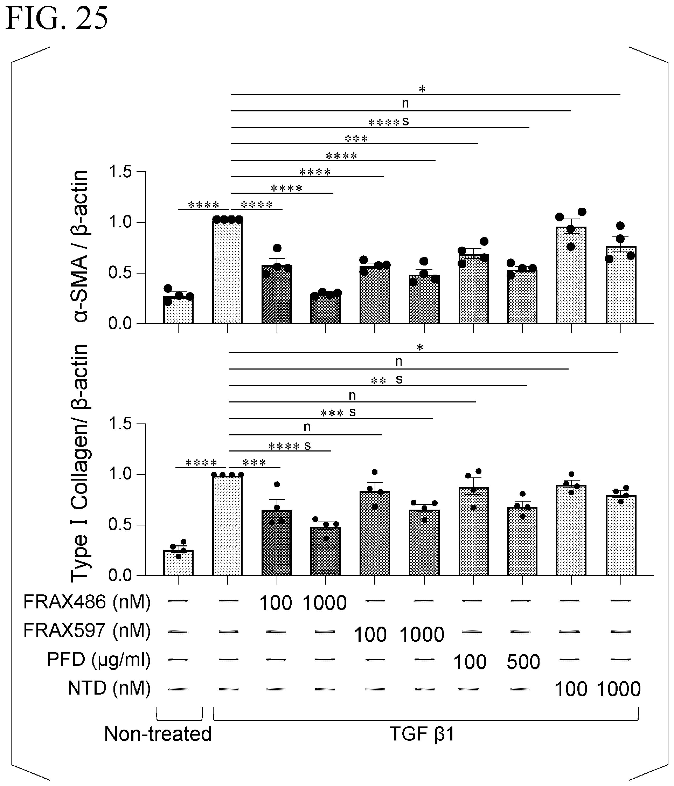

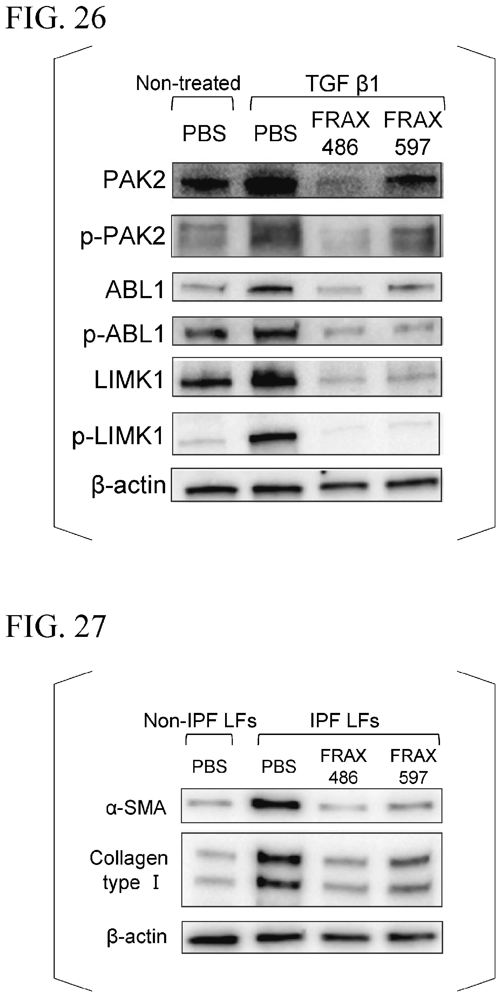

Next, in order to characterize the pharmacological antifibrotic properties targeting PAK2 from the viewpoint of IPF treatment, FRAX486 and FRAX597, which are PAK2 inhibitors, were selected and applied to a TGF-β-induced myofibroblast differentiation model of lung fibroblasts (see FIG. 24). FRAX486 and FRAX597 inhibit a plurality of PAK family members including PAK1, PAK2, and PAK3, but considering the expression pattern of PAK2 in IPF lung tissue, the main effect may be mediated through PAK2 (see FIG. 20). When treated with either FRAX486 or FRAX597, the TGF-β-induced myofibroblast differentiation was strongly suppressed based on the protein levels of α-smooth muscle actin (α-SMA) and type I collagen (see FIG. 24). These inhibitory effects were dose-dependent (see FIG. 25) and significantly exceeded the effects of two antifibrotic drugs, pirfenidone (PFD) and nintedanib (NTD), commonly used for IPF treatment. Immunofluorescence labeling of αSMA and type I collagen showed the inhibitory effects of FRAX486 and FRAX597 on TGF-β-induced myofibroblast differentiation. When the expression level of downstream proteins of PAK2 was examined using Western blotting (see FIG. 26), both inhibitors suppressed the expression and phosphorylation of PAK2, c-Abl, and LIMK1, but did not suppress the expression and phosphorylation of SMAD2/3. In particular, FRAX486 strongly inhibited the TGF-β-induced differentiation of lung fibroblasts into myofibroblasts. The transcriptional inhibition of downstream factors during the TGF-β-induced myofibroblast differentiation was confirmed by qPCR.

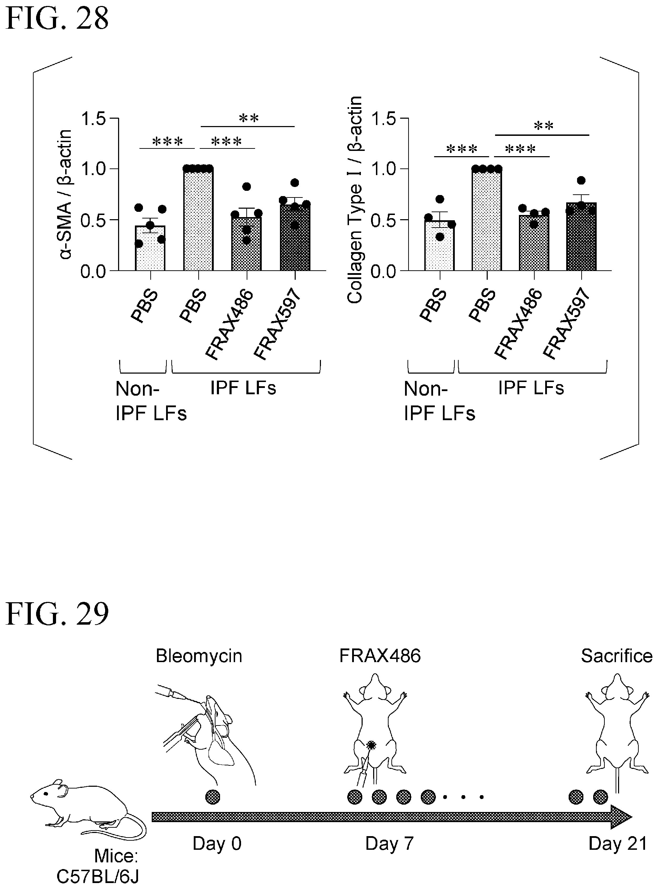

Next, the antifibrotic effects of FRAX486 and FRAX597 on lung fibroblasts isolated from IPF lungs were evaluated. The expression of α-SMA and type I collagen in IPF-derived fibroblasts was significantly reduced by treatment with these inhibitors (see FIGS. 27 and 28). Consistent with the TGF-β-induced myofibroblast differentiation model, the expression of PAK2 downstream proteins was significantly inhibited by FRAX486 and FRAX597 in IPF-derived fibroblasts. Furthermore, RT-PCR analysis confirmed the transcriptional inhibition of PAK2 downstream targets in IPF fibroblasts. The above results suggest that the antifibrotic effects of FRAX486 and FRAX597 are mediated through the inhibition of profibrotic differentiation in both the TGF-β-induced model and IPF-derived lung fibroblasts.

### [Therapeutic potential of PAK2 inhibitor in in vivo fibrosis model]

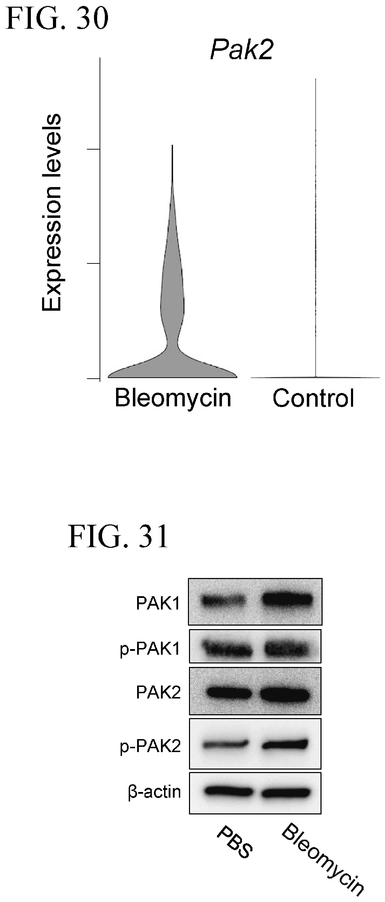

To examine the physiological antifibrotic effects of FRAX486 and FRAX597 in the progression of pulmonary fibrosis, a bleomycin (BLM)-induced pulmonary fibrosis mouse model was used to mimic the pathological features of human IPF (see FIG. 29). Before starting the experiment, the expression profile of PAK family members in BLM-induced mouse lungs was verified using a publicly available scRNA-seq dataset. Consistent with the findings in IPF lungs, an increase in PAK2 expression was observed in fibroblasts of BLM-induced lungs (see FIG. 30). On the other hand, the expression of PAK1 and PAK3 was not detected in these fibroblasts. Furthermore, the enhancement of PAK2 phosphorylation in lung tissue of the BLM-induced fibrosis model was confirmed, indicating activation during the progression of fibrosis (see FIG. 31).

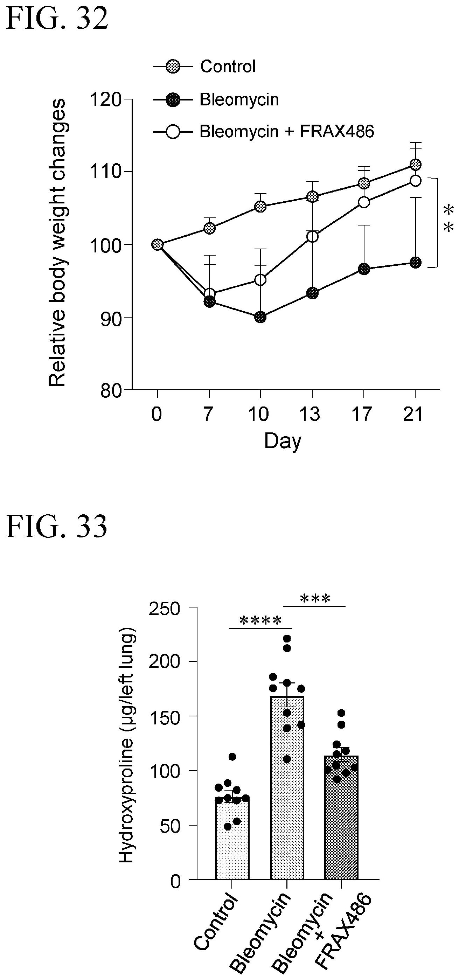

Based on the in vitro results showing that FRAX486 exhibited superior efficacy compared to FRAX597, FRAX486 was selected for further in vivo analysis. FRAX486 was administered intraperitoneally on the 7th day after BLM treatment, corresponding to start of the fibrotic state, and the therapeutic potential was evaluated (see FIG. 29). As shown in FIG. 32, severe weight loss was observed in BLM-treated mice compared to the weight loss observed in control mice. Surprisingly, treatment with FRAX486 restored the body weight of BLM-induced mice, with levels on day 21 comparable to the levels of control mice.

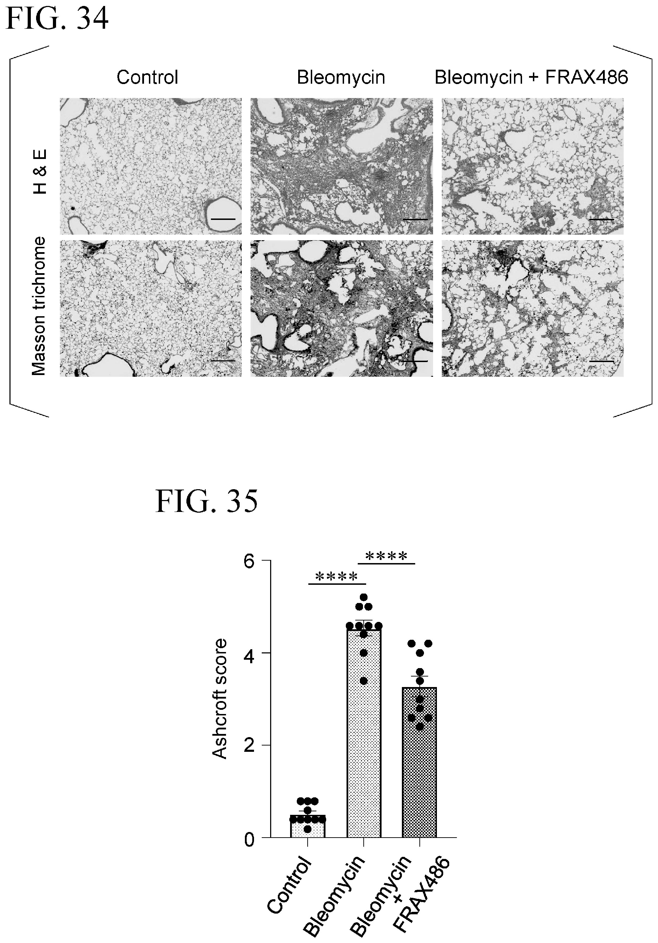

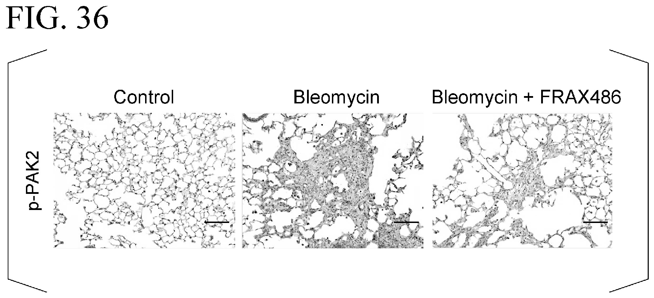

Histological analysis confirmed the antifibrotic effect of FRAX486. As confirmed by the hydroxyproline assay and Masson's trichrome staining results, intraperitoneal injection of FRAX486 significantly reduced the development of BLM-induced pulmonary fibrosis (see FIGS. 33 and 34). Further quantification using an Ashcroft score showed a significant decrease in fibrotic lesions (see FIG. 35). Immunohistochemical analysis also showed that FRAX486 treatment significantly inhibited the expression of phosphorylated PAK2 (p-PAK2) in the lungs of BLM-induced mice (see FIG. 36).

In summary, these findings demonstrate that, in a BLM-induced pulmonary fibrosis model, FRAX486 alleviates PAK2-mediated signal transduction and ameliorates pathological symptoms, supporting its potential as a therapeutic agent for IPF.

### INDUSTRIAL APPLICABILITY

According to the present invention, it is possible to provide a therapeutic agent for idiopathic pulmonary fibrosis based on a novel mechanism of action.

## Claims

1. A therapeutic agent for idiopathic pulmonary fibrosis, comprising a PAK inhibitor as an active ingredient.

2. A therapeutic agent for idiopathic pulmonary fibrosis, comprising a PAK2 inhibitor as an active ingredient.

3. The therapeutic agent for idiopathic pulmonary fibrosis according to Claim 1 or 2, wherein the therapeutic agent is intended for use in patients having WNT5A+ CTHRC1+ myofibroblasts in lung tissue.

4. A composition for treating idiopathic pulmonary fibrosis, comprising:
the therapeutic agent for idiopathic pulmonary fibrosis according to Claim 1 or 2; and
a pharmaceutically acceptable carrier.
